Europäisches Patentamt

European Patent Office

Office européen des brevets

(19)

(11) Publication number: **0 322 723**

**A2**

(12) **EUROPEAN PATENT APPLICATION**

(21) Application number: 88121341.7

(51) Int. Cl.⁴: **C12P 7/18 , C12P 7/58**

(22) Date of filing: **21.12.88**

(30) Priority: **25.12.87 JP 331258/87**

(43) Date of publication of application:
**05.07.89 Bulletin 89/27**

(84) Designated Contracting States:
**AT BE CH DE ES FR GB IT LI NL SE**

(71) Applicant: **FUJISAWA PHARMACEUTICAL CO., LTD.**
**3, Doshomachi 4-chome Higashi-ku**
**Osaka-shi Osaka 541(JP)**

(72) Inventor: **Ichikawa, Yoshio**
**No. 21-4, Imamiya 4-chome**
**Minoo-shi Osaka, 562(JP)**
Inventor: **Kitamoto, Yutaka**
**No. 137-18, Kumoyama**
**Tottori-shi Tottori, 680(JP)**
Inventor: **Kato, Nobuo**
**No. 106, Mihagino 3-chome**
**Tottori-shi Tottori, 680(JP)**
Inventor: **Mori; Nobuhiro**
**No. 110, Koyama-Nishi 4-chome**
**Tottori-shi Tottori, 680(JP)**

(74) Representative: **Türk, Gille, Hrabal**
**Bruckner Strasse 20**
**D-4000 Düsseldorf 13(DE)**

(54) **Preparation of gluconic acid and sorbitol.**

(57) A method of producing gluconic acid and sorbitol by subjecting glucose and fructose in aqueous solution to oxidation-reduction reaction in the presence of cells of a strain of microorganism of the genus Zymomonas wherein such cells are a dried cell fraction of a culture of the strain in a medium containing glucose as a carbon source, and a dried cell fraction of a culture of a bacterial strain of the genus Zymomonas in a medium containing glucose as a carbon source.

EP 0 322 723 A2

# PREPARATION OF GLUCONIC ACID AND SORBITOL

This invention relates to a novel method for producing gluconic acid or a salt thereof and sorbitol, and a dried cell fraction to be used therein.

There are several reports showing that when grown in a glucose- and fructose-containing medium or a sucrose-containing medium, Zymomonas such as Zymomonas mobilis elabolate not only ethanol as a main fermentation product but also some amount of sorbitol as a byproduct. Among such reports are there Liisa Viikari: Applied Microbiology and Biotechnology 19, 252-255 (1984), Liisa Viikari: Applied Microbiology and Biotechnology 20, 118-123 (1984), and Kevin D. Barrow, J. Grant Collins, Donald A. Leigh, Peter L. Rogers and Rhonda G. Warr: Applied Microbiology and Biotechnology 20, 225-232 (1984).

Recently Zachariou et al. isolated an enzyme called glucose-fructose oxidoreductase from cultured cells of Zymomonas mobilis [Michael Zachariou and Robert K. Scopes: Journal of Bacteriology 167, No. 3, 863-869 (1986)].

This enzyme occurs as tightly bound to the coenzyme NADP (nicotinamide adenine dinucleotide phosphate) and as such oxidizes glucose to gluconolactone and, at the same time, reduces the same mole of fructose as oxidized glucose to sorbitol.

However, when Zymomonas mobilis is cultured in a medium, its primary metabolic pathway from glucose or fructose to ethanol predominates, so that most of the glucose or fructose available in the medium is converted to ethanol. Therefore, in order that the metabolic system of Zymomonas mobilis may be utilized to convert glucose and fructose to gluconolactone and sorbitol and further convert the gluconolactone to gluconic acid to give gluconic acid and sorbitol as end products, it is necessary to first isolate glucose-fructose oxidoreductase which Zachariou discovered and gluconolactonase which hydrolyzes gluconolactone to gluconic acid from cultured cells of Zymomonas mobilis and then subject glucose and fructose to oxidation-reduction reaction in the presence of these enzymes. Therefore, the process is not efficient enough to be practiced on a commercial scale.

As a procedure for overcoming the above disadvantage, it may be contemplated to somehow block the enzyme system involved in the conversion of glucose or fructose to ethanol or find a mutant strain which does not elaborate the enzymes responsible for that conversion to ethanol. However, both are mere conceivable approaches and the inventors of this invention do not know of a report that such an attempt has ever proved successful.

The inventors of this invention explored the state of the art in this field with due diligence and found that by growing Zymomonas mobilis in a medium containing glucose as a carbon source, harvesting and drying the grown cells, and subjecting glucose and fructose in aqueous solution to oxidation-reduction reaction in the presence of the dried cells, the glucose in aqueous solution is oxidized to gluconic acid and, at the same time, the same mole of fructose as oxidized glucose is reduced to sorbitol and that there is little production of ethanol which is otherwise a major fermentation product with Zymomonas mobilis in a medium containing glucose and fructose. This invention has been conceived and developed from the above findings.

The experimentation by the inventors of this invention showed that when Zymomonas mobilis is grown in a medium containing glucose as a carbon source, the glucose in the medium is mostly converted to ethanol. This means that the grown cells as such contain a series of enzymes which convert glucose to ethanol.

However, if such cells were dried, a series of enzymes responsible for the conversion of glucose or fructose to ethanol appeared to be mostly inactivated while the aforesaid glucose-fructose oxidoreductase-NADP, which Zachariou isolated, and gluconolactonase which hydrolyzes gluconolactone to gluconic acid retained their activities.

It is not clear, indeed, why the mere drying procedure gives rise to such a phenomenon but it is a novel finding far beyond imagination for the conventional knowledge.

The medium for use in the production of dried cells of Zymomonas mobilis is virtually optional in kind only if it contains glucose and is suited to growth of Zymomonas mobilis. Since Zymomonas mobilis is an anaerobic bacterial species, it must be cultured by stationary culture or under anaerobic conditions, for example under degassing with nitrogen gas. Other cultural conditions such as incubation temperature and time may be similar to those used commonly for anaerobic culture.

For the drying of the resulting grown cells, either air drying or drying with a hydrophilic solvent such as acetone or a lower alcohol (e.g. methanol, ethanol, etc.) can be adopted. The air drying can be conducted at room temperature or under warming and the recommended temperature range is 10 to 60°C and preferably 20 to 40°C. Since the pH of the aqueous solution containing glucose and fructose drops gradually as the reaction proceeds to convert glucose to gluconic acid, it is recommended to control the reaction mixture at pH 5 to 8, preferably pH 6 to 7, by adding an aqueous solution of a base such as the hydroxide or carbonate of an alkali metal (e.g. sodium, potassium, etc.) or an alkaline earth metal (e.g. calcium, magnesium, etc.) or alternatively employ a suitable buffer solution.

In this reaction, glucose is oxidized to gluconic acid via gluconolactone and, at the same time, the same mole of fructose as oxidized glucose is converted to sorbitol. When the reaction system is comparatively rich in glucose, some glucose remains unreacted, while some fructose remains unreacted when the reaction system is rich in fructose. Such cases also fall within the scope of this invention.

From the resulting reaction mixture, gluconic acid or a salt thereof and sorbitol can be respectively isolated by the per se known procedure.

As described above, by growing Zymomonas mobilis in a medium containing glucose as a carbon source, harvesting and drying the grown cells and subjecting glucose and fructose in aqueous solution to oxidation-reduction reaction in the presence of the dried cells, the glucose and fructose can be simultaneously converted to gluconic acid or a salt thereof and sorbitol, respectively.

This invention will be described in further detail by way of the following test and working examples.

[Test Examples]

Test Example 1

Production of cultured cells, freeze-thawed cells, acetone-dried cells and air-dried cells of Zymomonas mobilis

(i) Cultured cells

In 1,000 ml of distilled water were dissolved 100 g of glucose, 5 g of yeast extract, 0.5 g of potassium dihydrogen phosphate, 0.5 g of magnesium sulfate (7 $H_2O$), 20 mg of ammonium ferrous sulfate (6 $H_2O$), 1 mg of biotin, and 2 mg of calcium pantothenate and the solution was adjusted to pH 7.0 with 1N-sodium hydroxide and sterilized at 115°C for 20 minutes to prepare a medium. (However, biotin and calcium pantothenate were filter-sterilized). A test tube (16.5 mm in diameter and 160 mm high) was charged with 5 ml of the above medium which was then inoculated with a loopful of Zymomonas mobilis ATCC 29191 grown in a deep-layer medium and stationary culture was carried out at 28°C for 36 hours. Then, a conical flask of 300 ml capacity was charged with 200 ml of the above medium, which was then inoculated with 0.3 ml of the seed culture prepared above and stationary culture was carried out at 28°C for 24 hours. After cultivation, the culture broth was centrifuged (10,000 G, 10 minutes) to harvest the cells, which were then washed with 2 portions of physiological saline.

Incidentally, Zymomonas mobilis ATCC 29191 is a bacterial strain available from the American Type Culture Collection (ATCC), a stock culture collection in the United States.

(ii) Freeze-thawed cells

The cultured cells obtained as above were suspended in a small quantity of physiological saline and the suspension was frozen at -20°C overnight and thawed at 40°C.

(iii) Acetone-dried cells

The cultured cells obtained as above were suspended in a small quantity of physiological saline and the suspension was dripped into acetone chilled to -30°C and stirred for a few minutes. The suspension was filtered through a glass filter and dried in a vacuum desiccator containing phosphorus pentoxide. The dried cells were comminuted in a mortar to provide a powder.

3

EP 0 322 723 A2

(iv) Air-dried cells

The cultured cells obtained as above were suspended in a small quantity of physiological saline and the suspension was spread on a petri dish and dried by the draft from an electric fan at room temperature (about 25°C). Then, the dried cells were further dehydrated in a vacuum desiccator containing phosphorus pentoxide and comminuted in a mortar to provide a powder.

Test Example 2

Oxidation-reduction of glucose and fructose with cultured cells, freeze-thawed cells, acetone-dried cells or air-dried cells of Zymomonas mobilis

Method

To 10 ml portions of a substrate solution containing 0.5 M glucose and 0.5 M fructose in McIlvaine buffer (pH 7.0) were added 60 mg (on a dry basis for cultured cells and freeze-thawed cells) each of the various cells prepared in Test Examples 1 (i) to (iv), respectively, and each mixture was incubated at 30°C with gentle shaking at 120 reciprocations/minute under degassing with nitrogen gas.

The concentrations of glucose, gluconic acid and fructose in the reaction mixture were monitored by the method of Sharp utilizing glucose oxidase and peroxidase (Sharp, P.: Clin. Clim. Acta. 40, 115-120, 1972) for glucose, the method of Mollening utilizing gluconate kinase and gluconate 6-phosphate dehydrogenase [Mollening, H., & Bergmeyer, H. U.: Method of Enzymatic Analysis, 1243-1247 (1974)] for gluconic acid, and the carbazole-cysteine-sulfuric acid method for fructose.

In this test, no assay was carried out for sorbitol.

Results

The results are shown in Table 1.

Table 1

| Cells | Reaction time (hours) | Product gluconic acid (M) | Residual glucose (M) | Residual fructose (M) | pH | Yield of gluconic acid based on glucose |
|---|---|---|---|---|---|---|
| Cultured cells | 0 | - | 0.5 | 0.5 | - | - |
| | 2 | 0.065 | 0.369 | 0.405 | 5.6 | 50 |
| | 4 | 0.077 | 0.285 | 0.388 | 4.9 | 36 |
| | 6 | 0.079 | 0.246 | 0.372 | 4.6 | 31 |
| Freeze-thawed cells | 0 | , - | 0.5 | 0.5 | 7.0 | - |
| | 2 | 0.080 | 0.368 | 0.425 | 5.0 | 61 |
| | 4 | 0.106 | 0.344 | 0.406 | 4.5 | 68 |
| | 6 | 0.122 | 0.320 | 0.388 | 4.3 | 68 |
| Acetone-dried cells | 0 | - | 0.5 | 0.5 | 7.0 | - |
| | 2 | 0.101 | 0.397 | 0.406 | 5.0 | 98 |
| | 4 | 0.128 | 0.372 | 0.393 | 4.6 | 100 |
| | 6 | 0.139 | 0.350 | 0.368 | 4.3 | 93 |
| Air-dried cells | 0 | - | 0.5 | 0.5 | 7.0 | - |
| | 2 | 0.096 | 0.389 | 0.401 | 5.1 | 87 |
| | 4 | 0.123 | 0.368 | 0.379 | 4.5 | 93 |
| | 6 | 0.134 | 0.344 | 0.378 | 4.3 | 86 |

4

It is apparent from the above test examples that the acetone-dried cells and air-dried cells are suitable for the purposes of this invention.

Example 1

Production of air-dried cells of Zymomonas mobilis

The procedure described in Test Example 1 was followed to provide 240 mg of air-dried cells of Zymomonas mobilis.

Example 2

Oxidation-reduction of glucose and fructose with air-dried cells of Zymomonas mobilis

Method

To 10 ml of the substrate-McIlvaine buffer (pH 7.0) solution described in Test Example 2 were added 60 mg of the dried cells obtained in Example 1 and the mixture was incubated at 30°C with gentle stirring while the reaction system was controlled at pH 6.7 with 5N sodium hydroxide using a pH-stat.

The concentrations of glucose, gluconic acid, fructose and sorbitol in the reaction mixture were monitored by the methods described in Test Example 2 for glucose, gluconic acid and fructose and the method of Bergmyer utilizing sorbitol dehydrogenase [Bergmyer, H. U., Gruber, W. & Gutmann, I.: Methods of Enzymatic Analysis, 1323-1326 (1974)] for sorbitol.

Results

The results are shown in Table 2.

Table 2

| Reaction time (hours) | Product gluconic acid (M) | Residual glucose (M) | Product sorbitol (M) | Residual fructose (M) |
|---|---|---|---|---|
| 0 | - | 0.5 | - | 0.5 |
| 1 | 0.067 | 0.349 | 0.083 | 0.372 |
| 2 | 0.110 | 0.246 | 0.123 | 0.241 |
| 6 | 0.319 | 0.105 | 0.396 | 0.121 |
| 8 | 0.344 | 0.056 | 0.368 | 0.057 |
| 10 | 0.411 | 0.035 | 0.499 | 0.042 |

Zymomonas mobiles ATCC 29191 and other strains of Zymomonas having the above described activities have been deposited by other parties before the priority date of this invention and are commercially available. By tests described in the mentioned prior art and this specification it is easily possible for the one skilled in the art to find out whether a strain of microorganism of the genus Zymomonas has glucose-fructose oxidoreductase, i.e. converts glucose and fructose to gluconolactone and sorbitol, and further converts the gluconolactone to gluconic acid. Cells, which are obtained by growing such microorganism in a medium containing glucose as a carbon source, harvesting and drying the grwon cells, are useful according to this invention.

**Claims**

1. A method of producing gluconic acid and sorbitol by subjecting glucose and fructose in aqueous solution to oxidation-reduction reaction in the presence of cells of a strain of microorganism of the genus Zymomonas characterized in that such cells are a dried cell fraction of a culture of the strain in a medium containing glucose as a carbon source.

2. The method of producing glucose and sorbitol of Claim 1 in which the genus Zymomonas is Zymomonas mobilis.

3. A dried cell fraction of a culture of a bacterial strain of the genus Zymomonas in a medium containing glucose as a carbon source.

4. The dried cell fraction of Claim 3 in which the genus Zymomonas is Zymomonas mobilis.